# EUROPEAN PATENT SPECIFICATION

(11) **EP 0 789 796 B1**
(45) Date of publication and mention of the grant of the patent: **11.10.2000**
(21) Application number: 95910572.7
(22) Date of filing: 09.03.1995
(51) Int. Cl.: D06M 13/432, A61L 15/16, D01F 2/10

(54) **FIBROUS STRUCTURE CONTAINING UREA PEROXIDE AND METHOD FOR ITS MANUFACTURE**
HARNSTOFFPEROXIDE ENTHALTENDE FASERSTRUKTUR UND VERFAHREN ZU DESSEN HERSTELLUNG
STRUCTURE FIBREUSE RENFERMANT DU PEROXYDE D'UREE ET PROCEDE POUR SA FABRICATION

(30) Priority: 09.03.1994 FI 941126
(43) Date of publication of application: 20.08.1997
(73) Proprietor: Parén, Aarto, 37620 Valkeakoski (FI); Vapaaoksa, Pekka, 37600 Valkeakoski (FI); Mustakallio, Kimmo, 00170 Helsinki (FI)
(72) Inventor: Parén, Aarto, 37620 Valkeakoski (FI); Vapaaoksa, Pekka, 37600 Valkeakoski (FI); Mustakallio, Kimmo, 00170 Helsinki (FI)
(74) Representative: Hakola, Unto Tapani
(86) International application number: FI9500129
(87) International publication number: WO9524525

(56) References cited:
- EP-A- 0 268 459
- WO-A-93/13249
- SE-B- 417 321
- US-A- 4 033 913

## Description

The invention relates to a fibrous structure containing urea peroxide and to a method for its manufacture.

As it has been known, urea peroxide can be used as oxygen source in rather many different fields of use. It is known that urea peroxide has a retarding effect on the growth of some bacteria. It also prevents the growth of harmful fungi, such as mould. Urea peroxide has been used as deodorant for removing for example odours created by sulphur compounds. It absorbs among other things formaldehyde, phenol, hydrogen sulphide and carbon disulphide.

Urea peroxide is water-soluble, and it is consequently dissolved under moist conditions and starts to decompose already at a temperature of ca. 40°C. Usually urea peroxide absorbed in a fabric or fibre will at once dissolve in water from the fabric. Because of the solubility and low decomposing temperature, the effects of urea peroxide absorbed in the fabric will have a short period of action, which restricts the possibilities to use such textiles. This fact is true also for viscose materials, which are generally used for hospital and hygiene purposes.

The purpose of the invention is to bring about a decisive improvement as far as the above-mentioned drawbacks are concerned. For realizing this purpose, the fibrous structure according to the invention is characterized in that the fibre is polysilicic acid containing fibre having the urea peroxide absorbed therein. Correspondingly, the method according to the invention is characterized in that a polysilicic acid containing fibre is treated with a solution containing urea peroxide for causing the fibre to absorb urea peroxide.

The fibrous structure comprises a supporting structure and polysilicic acid bound thereto. The supporting structure can be of cellulose, preferably regenerated viscose cellulose, in which the polysilicic acid is homogeneously distributed in the same phase.

The fibrous structure can be in any product form, such as in the form of individual fibres or a structure made of fibres, such as a textile structure, e.g. non-woven fabric. The urea peroxide can be caused to be absorbed already by fibres, whereafter the fibres can be assembled into the textile structure, or it can be absorbed as late as by the textile structure made of the fibres.

As the behaviour of urea peroxide with viscose fibre containing the polysilicic acid and viscose fibre containing aluminium silicate (Visil and Visil AP, respectively) was examined, it was surprisingly discovered that the dissolution of urea peroxide decreased essentially, and the antibacterial and disinfecting efficiency of the textile was prolonged essentially. In antibacterial tests, it was discovered that a textile containing urea peroxide formed around itself a protection periphery, inside which no bacteria were present.

A textile containing significant amounts of oxidizing compounds is also very inflammable in normal cases. An inorganic textile part containing polysilicic acid or aluminium silicate is known to be flame-retarding. For this reason, considerably larger urea peroxide amounts can be safely used compared with normal textiles.

The amount of the urea peroxide is adjusted in the Visil-textile by adjusting the concentration of the impregnating solution, the ratio of textiles to the amount of solution and/or their retention times in the solution. For the treatment, the most suitable concentration of the solution is between 5 and 250 g/l urea peroxide.

The impregnation can be effected by immersion or spraying. The urea peroxide amount remaining in the fibre depends on the textile structure, absorbency and water retention capacity. A suitable amount of urea peroxide in a ready-made fibrous structure can be 0.1 to 10 wt.% of fibre dry weight, depending on the field of use.

The method of manufacture and properties of the polysilicic acid containing viscose and the aluminium silicate containing viscose are described e.g. in GB-A-1 064 271 and Finnish Patent Application Fi-A-916187 (WO-A-93/13249).

In research which led to the invention disclosed in the above-mentioned patent application, it was discovered that the Visil fibre behaves chemically in the same way as the polysilicic acid can be expected to behave in hydrogel forms (Iler, "Chemistry of Silica", p. 622-714, Wiley, New York, 1979). This affords the possibility to bind various metal cations and nitrogen containing ions, ammonium ions or quaternary ammonium compounds to the fibre.

Antibacterial and biocidic properties can be provided in the fibre by binding any known antibacterial substances in the fibre. Textiles with an antibacterial finish have been developed quite many during the last few years, and an exhaustive list can be found in the presentation by M. Burgeois, "Fibres with Antiseptic Action", INDEX -93, Congress Papers, Edana, Geneva, 1993. However, the use of antibacterial textiles has not become established in the treatment of wounds, because they will have a considerably high price owing to expensive applying methods or costs of chemicals. The use of some products is also limited by toxicity, smell or colour.

In the urea peroxide containing fibrous structure according to the invention, these drawbacks have been eliminated. Because of the chemical properties of the Visil fibre, the bonding of the urea peroxide to the fibre is easy and for example the electrochemical pretreatment described in the above-mentioned publication is not needed. Urea peroxide is a cheap and well-known disinfecting agent. Colour and odour problems do not occur. Further, owing to its bleaching and other chemical properties, the field of use of the textile materials containing urea peroxide provides a larger market than the use in hospitals, which also is a cost-lowering factor. Such other types of use can be the bleaching of hair or teeth, deodorizing filters and disinfecting towels. For example in hair bleaching, it is possible to use the fibrous structure in the form of a non-woven piece, which can be attached to hairs where the bleaching is desirable when making stripes for example. When the urea peroxide ends up in suitable environment where it can be exchanged with another substance, the urea peroxide is then liberated from the structure and causes the desired effect.

For the purpose of waste treatment, the product according to the invention is also harmless, because in course of composting, it will decompose as the temperature exceeds 40°C and it will turn into part of nutrient-rich compost.

The Visil fibres intended for structures according to the invention have been tested by the manufacturer and were found not to cause skin irritation or allergy as such. The guarantees of the manufacturer are not valid in these respects, if other fibres or chemicals susceptible of leaving harmful substances in the fibre are used in the further processing. A man skilled in the art of textiles and fibres can in any case prepare a fibre or textile structure where the fibres remain clean.

On the other hand, it is also known that urea peroxide has been used for medical and skin care purposes where it is harmless if used correctly. In the product according to the invention, it is bound to the fibres, from which it under normal conditions will be slowly released, which for its part decreases the risk of overdose and potential problems associated therewith.

In a dry state, the urea peroxide is a white crystalline substance, which dissolves in water and alcohols. The substance decomposes at ca. from 75 to 78°C, under the influence of moisture already at ca. 40°C, producing urea, water and oxygen. Urea peroxide can be used among other things as a source of anhydrous hydrogen peroxide, and in dry state it contains ca. 36% H₂0₂. The invention thus makes it possible to store the hydrogen peroxide dry in a fibrous structure. In a literature search, urea peroxide was found to have applications among other things in bleaching of fibres, in cosmetic products (permanent waves etc.), in synthesis reagents, etc. Because the urea peroxide dissolves in water and can be preserved dry, a dry-storable product can be manufactured by impregnating fibre with its water solution. Hydrogen peroxide is released from the product when it is placed in a suitable environment, such as on top of a wound. Such products could be utilized for example as wound bandages, etc.

Urea peroxide as such does not require polysilicic acid or Visil fibre in order to be effective or be retained in the fibre. Instead of viscose fibres, also any water-absorbing fibre can be used. However, in practice, urea peroxide seems to be best preserved by Visil fibre for some reason. Also, it is not washed out so easily from Visil fibre as for example from viscose, which will be verified in the following example.

### Example

In the tests, normal viscose fibre Avihaf SD, polysilicic acid containing viscose fibre Visil and polysilicic acid containing viscose fibre Visil AP, which contains aluminium silicate groups, were used as fibres.

CO(NH₂)₂ · H₂0₂ was prepared by crystallizing in hydrogen peroxide solution of urea (H₂0₂ = 36.8%). From this solution, a 60 g/l solution (21.5 g/l H₂0₂) was prepared in cold water (20°C). The treatment of the samples was the following:

CO(NH₂)₂ · H₂0₂ solution 20°C, 5 min, liquor ratio 1:30 → squeezed to a moisture of 200% → A-samples dried (drum dryer without steam), B-samples wash in 20°C water, liquid ratio 1:100 → squeezing to a moisture of 200% → drying in a drum dryer without steam.

Hydrogen peroxide was titrated from the samples on the following day, and the results are shown in Table 1, where the names of the fibres are followed by dtex values and lengths in mm.

**Table 1**

| **Sample** | | **H**_{**2**}**0**_{**2**} **%** |
|---|---|---|
| Avihaf SD 1.7/40 | A | 1.11 |
| Avihaf SD 1.7/40 | B | 0.08 |
| Visil 2.2/50 | A | 0.64 |
| Visil 2.2/50 | B | 0.36 |
| Visil AP 3.5/50 | A | 0.88 |
| Visil AP 3.5/50 | B | 0.41 |

For some reason, the Visil fibre and Visil AP fibre retained more substance after washing with water. Avihaf SD fibre retained the most in impregnation owing to its better absorbent properties, but the substance was removed almost totally by only rinsing with water. The substance seemed also to be best retained in the Visil fibre.

The samples were kept on the table at 22°C in relative humidity of ca. 60%, and H₂0₂ was titrated daily. The contents remained almost unchanged up to three days. Table 2 shows the H₂0₂ concentrations in the samples of Table 1 after four days had elapsed.

**Table 2**

| **Sample** | **H**_{**2**}**0**_{**2**} **%** |
|---|---|
| Avihaf SD/A | 0.37 |
| Avihaf SD/B | 0 |
| Visil PV/A | 0.25 |
| Visil PV/B | 0.31 |
| Visil AP/A | 0.43 |
| Visil AP/B | 0.38 |

### Mould test

Accelerated mould tests were performed on the fibres by soaking the sample pieces by a mould-nutrient solution made in distilled water. A mould strain was added, the incubation at 37°C was started, and the dishes were read at predetermined intervals. There was no growth in the fibres containing urea peroxide during one week, whereafter the mould growth started. On the contrary, the fibre which did not contain urea peroxide showed growth to such extent that the dish was covered with mould as soon as after only one day.

### Antibacterial test

The following is a short description of tests conducted on the urea peroxide containing fibre with *Pseudomonas aeruginosa* ATCC 27853.

One eyelet of bacterial culture was taken from a *Pseudomonas aeruginosa* culture, and it was mixed with ca. 3 ml of sterile 0.9% NaCl solution. The tube was shaken well for distributing the bacterial mass to a uniform suspension (the liquid was turbid in visual examination). This suspension was poured on a nutrient agar dish, and the dish was rotated to distribute the suspension uniformly over the whole dish. It was placed in an incubator, 30°C, for a moment, and then the suspension was poured away from the dish. "Tablets" of 7 mm diameter, punched out from Visil non-woven fabric, were placed on the dishes, and thereafter 80 µl of urea peroxide solution was pipetted by means of a "Finnpipet" pipette on them in different concentrations (15% to 3%), a tablet with bare urea as a control. The dishes were incubated in the incubator (30°C) overnight. The urea peroxide produced strong and clear inhibition rings around the fibrous tablet containing urea peroxide, but not around the urea tablet. The inhibition rings remained clear during several weeks in a refrigerator.

Corresponding tests were performed with *Staphylococcus aureus* ATCC 25923 and *Xanthomonas maltophilia.* The inhibition rings were detectable already in concentrations of from 1 to 3%.

Also other fibres where polysilicic acid is bound can be used in the invention, for example synthetic fibres. For example, synthetic fibres containing polysilicic acid are known where the supporting structure is polyester. The competitive price of the polysilicic acid containing viscose fibre makes it, however, the most advantageous alternative.

## Claims

1. A urea peroxide containing fibrous structure, which contains urea peroxide absorbed therein, **characterized** in that the fibre is polysilicic acid containing fibre having the urea peroxide absorbed therein.

2. A fibrous structure according to claim 1, **characterized** in that the supporting structure of the fibrous structure is cellulose, especially regenerated viscose cellulose, in which the polysilicic acid is homogeneously distributed in the same phase.

3. A fibrous structure according to claim 1 or 2, **characterized** in that it contains urea peroxide from 0.1 to 10 wt.% of the dry weight of the fibre.

4. A method for manufacture of a urea peroxide containing fibrous structure, in which method urea peroxide is absorbed in the fibre, **characterized** in that a fibre containing polysilicic acid bound to its supporting structure is treated with a solution containing urea peroxide for causing the fibre to absorb urea peroxide.

5. A method according to claim 4, **characterized** in that the concentration of the impregnating solution is in the range of from 5 to 250 g/l urea peroxide.

## Patentansprüche

1. Harnstoffperoxid enthaltende Faserstruktur, die Harnstoffperoxid in ihr absorbiert enthält, dadurch gekennzeichnet, dass die Faser eine Polykieselsäure enthaltende Faser ist, in der das Harnstoffperoxid absorbiert ist.

2. Faserstruktur nach Anspruch 1, dadurch gekennzeichnet, dass die Trägerstruktur der Faserstruktur Zellulose, insbesondere regenerierte Viskose-Zellulose, ist, in der die Polykieselsäure in derselben Phase homogen verteilt ist.

3. Faserstruktur nach Anspruch 1 oder 2, dadurch gekennzeichnet, dass sie Harnstoffperoxid zwischen 0.1 und 10 Gew.% des Trockengewichts der Faser enthält.

4. Verfahren zur Herstellung einer Harnstoffperoxid enthaltenden Faserstruktur, bei dem das Harnstoffperoxid in der Faser absorbiert wird, dadurch gekennzeichnet, dass eine Faser, die Polykieselsäure gebunden an seine Trägerstruktur enthält, mit einer Harnstoffperoxid enthaltenden Lösung behandelt wird, um eine Absorption des Harnstoffperoxids in der Faser zu bewirken.

5. Verfahren nach Anspruch 4 dadurch gekennzeichnet, dass die Konzentration der Imprägnierlösung im Bereich von 5 bis 250 g/l Harnstoffperoxid liegt.

## Revendications

1. Structure fibreuse contenant du peroxyde d'urée qui contient du peroxyde d'urée absorbé à l'intérieur, caractérisée en ce que la fibre est une fibre contenant du poly(acide silicique) ayant le peroxyde d'urée absorbé à l'intérieur.

2. Structure fibreuse selon la revendication 1, caractérisée en ce que la structure de support de la structure fibreuse est de la cellulose, en particulier de la cellulose de type viscose régénérée où le poly(acide silicique) est distribué dans la même phase de manière homogène.

3. Structure fibreuse selon la revendication 1 ou 2, caractérisée en ce qu'elle contient 0,1 à 10% en masse de peroxyde d'urée par rapport à la masse sèche de la fibre.

4. Procédé pour fabriquer une structure fibreuse contenant du peroxyde d'urée, procédé dans lequel du peroxyde d'urée est absorbé dans la fibre, caractérisé en ce qu'une fibre qui contient du poly(acide silicique) lié à sa structure de support est traitée avec une solution contenant du peroxyde d'urée pour amener la fibre à absorber le peroxyde d'urée.

5. Procédé selon la revendication 4, caractérisé en ce que la concentration de la solution d'imprégnation est dans la gamme de 5 à 250 g/l de peroxyde d'urée.
